(19) Europäisches Patentamt
European Patent Office
Office européen des brevets

(11) Veröffentlichungsnummer: **0 049 398**
**B1**

# (12) EUROPÄISCHE PATENTSCHRIFT

(45) Veröffentlichungstag der Patentschrift: **06.03.85**

(51) Int. Cl.⁴: **C 07 D 265/20**, A 01 N 43/86

(21) Anmeldenummer: **81107364.2**

(22) Anmeldetag: **17.09.81**

(54) **4H-3,1-Benzoxazinderivate, Verfahren zu ihrer Herstellung und ihre Verwendung zur Bekämpfung unerwünschten Pflanzenwuchses.**

(30) Priorität: **08.10.80 DE 3037970**

(43) Veröffentlichungstag der Anmeldung:
**14.04.82 Patentblatt 82/15**

(45) Bekanntmachung des Hinweises auf die Patenterteilung:
**06.03.85 Patentblatt 85/10**

(84) Benannte Vertragsstaaten:
**AT BE CH DE FR GB IT LI LU NL SE**

(56) Entgegenhaltungen:
**EP - A - 0 017 931**
**EP - A - 0 024 669**
**FR - A - 2 121 341**
**SU - A - 178 380**
**US - A - 3 357 977**
**US - A - 3 914 121**

**JOURNAL OF THE CHEMICAL SOCIETY, (C), 1968, Seite 1594 D.I. BAIN et al.: "Synthesis of 2-substituted-4H-3,1-benzoxazin-4-ones"**
**CHEMICAL ABSTRACTS, Band 70, Nr. 4, 27. Januar 1969, Seite 65, Nr. 20992y, Columbus, Ohio, U.S.A.**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Hamprecht, Gerhard, Dr., Rote-Turm-Strasse 28, D-6940 Weinheim (DE)**
Erfinder: **Wuerzer, Bruno, Dr. Dipl.-Landwirt, Ruedigerstrasse 13, D-6701 Otterstadt (DE)**

**EP 0 049 398 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft 4H-3,1-Benzoxazinderivate, ein Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

Substituierte 4H-3,1-Benzoxazin-4-one sind als Zwischenprodukte für die Synthese pharmakologisch wirksamer Verbindungen (DE-A-1 670 375) bekannt. Außerdem ist bekannt, daß 4H-3,1-Benzoxazinonderivate herbizid wirksam sind, so z. B. 4H-3,1-Benzoxazin-4-one, die im Phenylteil durch Halogen, Nitro oder Alkyl substituiert sind (DE-A-2 556 590), halogensubstituierte 4H-3,1-Benzoxazin-4-one, die in 2-Stellung einen gegebenenfalls substituierten Phenylrest tragen, wobei allerdings nur unsubstituierte 4H-3,1-Benzoxazinderivate beschrieben sind (BE-A-648 259), sowie 2-Phenyl-4H-3,1-benzoxazin-4-one, deren Phenylsubstituent in m- oder p-Stelung durch Halogen oder Trifluormethyl substituiert ist (US-A-3 914 121).

Es wurde gefunden, das 4H-3,1-Benzoxazinderivate der Formel I

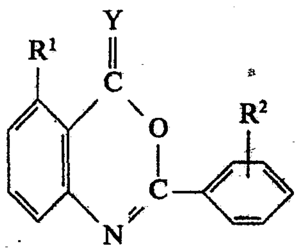

(I)

in der

Y Sauerstoff oder Schwefel und
$R^1$ Halogen bedeutet, wenn $R^2$ Halogen in m- oder p-Stelung ist, oder
$R^1$ Methyl bedeutet, wenn $R^2$ Fluor, Trifluormethyl, Trifluormethoxy oder Chlordifluormethoxy in m-Stellung ist, oder
$R^1$ Nitro bedeutet, wenn $R^2$ Trifluormethoxy oder Chlordifluormethoxy in m-Stellung ist,

bei guter Verträglichkeit für eine Reihe von Kulturpflanzen herbizid wirksam sind.

In der Formel I bedeuten $R^1$ beispielsweise Fluor, Chlor, Brom, Jod, Nitro oder Methyl, $R^2$ beispielsweise Fluor, Chlor, Brom, Jod, Trifluormethyl, Trifluormethoxy oder Chlordifluormethoxy.

Bevorzugte Verbindngen der Formel I sind solche, bei denen Y für Sauerstoff und $R^1$ für Halogen, insbesondere für Fluor oder Chlor, stehen, wenn $R^2$ Halogen, insbesondere Fluor oder Chlor, in m- oder p-Stellung am Phenylring ist.

Man erhält die 4H-3,1-Benzoxazinderivate der Formel I durch Umsetzung einer substituierten Anthranilsäure der Formel II

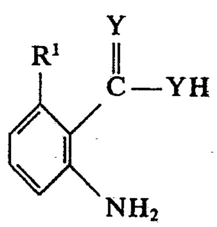

(II)

in der
$R^1$ und Y die obengenannten Bedeutungen haben, mit mindestens dem zweifachen molaren Überschuß eines Carbonsäurehalogenids der Formel III

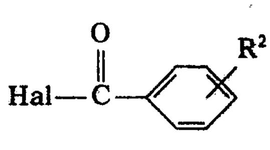

(III)

in der
$R^2$ die obengenannten Bedeutungen hat und Hal für Halogen, insbesondere Fluor, Chlor oder Brom, steht, in einem aromatischen tertiären Amin als Lösungsmittel bei einer Temperatur im Bereich zwischen 10 und 60° C.

Verwendet man 6-Chloranthranilsäure und 3-Fluor-benzoylchlorid als Ausgangsmaterialien, so kann der Reaktionsablauf durch folgendes Formelschema wiedergegeben werden:

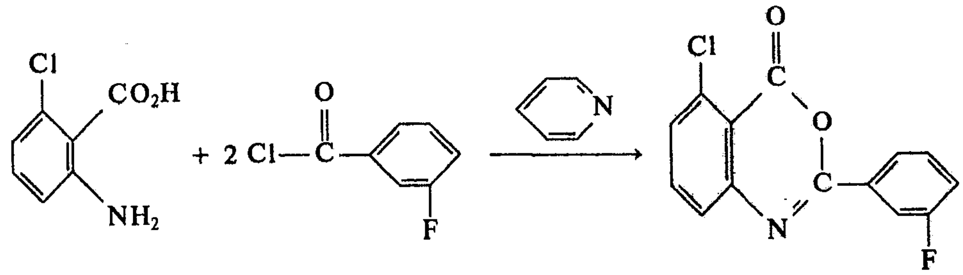

Zweckmäßigerweise läßt man einen zweifachen Überschuß des Carbonsäurehalogenids der Formel III in eine Lösung der Anthranilsäure der Formel II in der 5- bis 25fachen molaren Menge eines aromatischen Amins, bezogen auf Anthranilsäure, bei einer Temperatur zwischen 10 und 60° C einlaufen und rührt dann 30 Minuten bei 25° C nach (J. Chem. Soc. (C) 1968, 1593). Zur Aufarbeitung kann dann Eiswasser eingerührt und vom ausgefallenen Niederschlag abgesaugt werden. Ebenso kann das Carbonsäurehalogenid der Formel III vorgelegt werden.

Geeignete aromatische tertiäre Amine sind beispielsweise Pyridin, $\alpha$-, $\beta$-, $\gamma$-Picolin, Lutidin, Chinolin und Acridin.

Die Benzoxazinderivate der Formel I können auch dadurch erhalten werden, daß man eine substituierte Anthranilsäure der Formel II

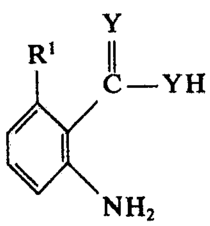

(II)

in der
$R^1$ und Y die oben angegebenen Bedeutungen haben, oder ein Alkalimetall- oder Erdalkalimetallsalz dieser Anthranilsäure mit ungefähr stöchiometrischen Mengen eines Carbonsäurehalogenids der Formel III

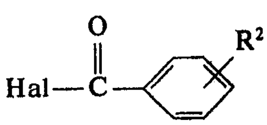

(III)

in der
$R^2$ die im Anspruch 1 genannten Bedeutungen hat und Hal für Halogen steht, in einem inerten organischen Lösungsmittel oder in Wasser und gegebenenfalls in Anwesenheit eines Säureakzeptors bei einer Temperatur im Bereich von 0 und 60° C zu einem Carbonamid der Formel IV

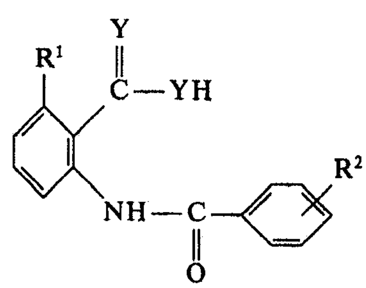

(IV)

in der
$R^1$, $R^2$ und Y die obengenannten Bedeutungen haben, umsetzt und dieses dann in Gegenwart eines wasserentziehenden Mittels bei einer Temperatur zwischen 30 und 150° C cyclisiert.

Verwendet man 3-Trifluormethylbenzoylfluorid und 6-Methylanthranilsäure als Ausgangsmaterialien, so kann der Reaktionsablauf durch folgendes Formelschema wiedergegeben werden:

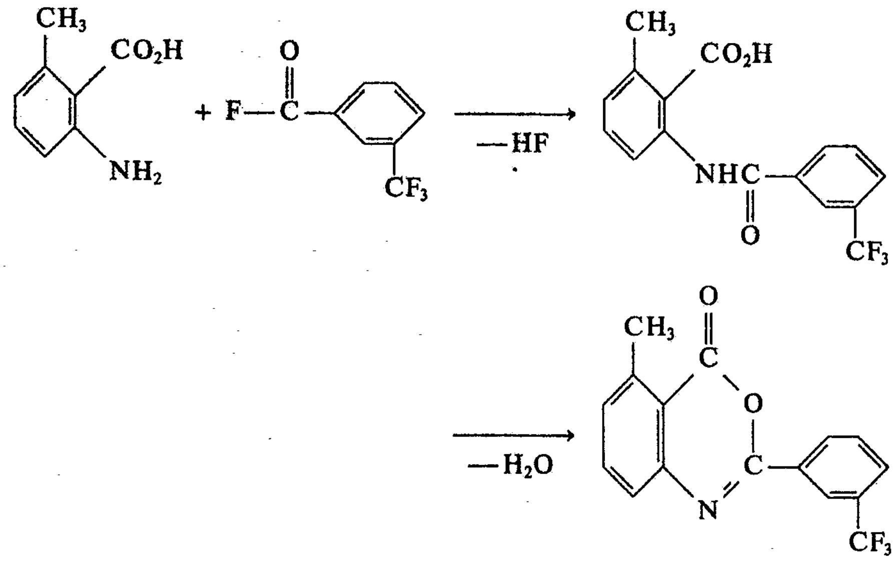


Geeignete inerte Lösungsmittel für diese Umsetzung sind Kohlenwasserstoffe, wie Ligroin, Benzin, Toluol, Pentan, Hexan, Cyclohexan, Petrolether, Halogenkohlenwasserstoffe, wie Methylenchlorid, Chloroform, Tetrachlorkohlenstoff, 1,1- und 1,2-Dichlorethan, 1,1,1- und 1,1,2-Trichlorethan, Chlorbenzol, o-, m-, p-Dichlorbenzol, o-, m-, p-Chlortoluol, Nitrokohlenwasserstoffe, wie Nitrobenzol, Nitroethan, o-, m-, p-Chlornitrobenzol, Nitrile, wie Acetonitril, Butyronitril, Isobutyronitril, Ether, wie Diethylether, Di-n-propylether, Tetrahydrofuran, Dioxan, Ester wie Acetessigester, Ethylacetat oder Isobutylacetat, oder Amide, wie Formamid, Methylformamid oder Dimethylformamid.

Als Säureakzeptoren können alle üblichen Säurebindemittel verwendet werden. Hierzu gehören vorzugsweise Alkalihydroxide, Alkalicarbonate und tertiäre organische Basen. Besonders geeignet sind beispielsweise Natriumhydroxid, Natriumcarbonat, Natriumhydrogencarbonat, Triethylamin, Pyridin, Trimethylamin, α-, β-, γ-Picolin, Lutidin, N,N-Dimethylanilin, N,N-Dimethylcyclohexylamin, Chinolin, Tri-n-propylamin und Tri-n-butylamin. Das Säurebindemittel wird zweckmäßigerweise in äquivalenten Mengen, bezogen auf Carbonsäurehalogenid der Formel III, eingesetzt.

Als wasserentziehende Mittel können symmetrische und gemischte Carbonsäureanhydride, wie Essigsäureanhydrid, Propionsäureanhydrid, Buttersäureanhydrid, Ameisensäureessigsäureanhydrid, Ameisensäurepropionsäureanhydrid, oder Essigsäurepropionsäureanhydrid, ferner Dicyclohexylcarbodiimid oder Thionylchlorid verwendet werden. Die Cyclisierung wird unter Zusatz der 1- bis 10fachen molaren Menge an wasserentziehendem Mittel, bezogen auf Carbonamid der Formel IV, durchgeführt.

Die Ausgangsstoffe der Formeln II und III werden in ungefähr stöchiometrischem Verhältnis eingesetzt, d. h. zweckmäßigerweise in einem Unter- bzw. Überschuß von bis zu 10% Ausgangsstoff der Formel III gegenüber Ausgangsstoff der Formel II.

Zweckmäßigerweise wird das Verfahren so durchgeführt, daß man über zwei Zuführungen das Carbonsäurehalogenid der Formel III und die äquivalente Menge an Säureakzeptor bei einer Temperatur zwischen 0 und 60° C zu einer ungefähr äquivalenten Menge der Anthranilsäure der Formel II bzw. ihres Salzes in einem inerten organischen Lösungsmittel bzw. in Wasser zulaufen läßt. Dann rührt man 15 Minuten bei Raumtemperatur nach. Das Reaktionsgemisch wird dann gegebenenfalls eingeengt, in der Wärme mit 5 n Salzsäure angesäuert, abgekühlt und abgesaugt (J. Org. Chem. 2, 396 (1944)), wobei eine N-Acyl-2-aminobenzoesäure erhalten wird. Diese kann dann in Gegenwart der 5- bis 10fachen molaren Menge an Carbonsäureanhydrid als wasserentziehendem Mittel, beispielsweise Acetanhydrid, durch Rühren — gegebenenfalls unter Abdestillation der entstandenen Essigsäure — bei einer Temperatur zwischen 30 und 150° C zu dem gewünschten 4H-3,1-Benzoxazin cyclisiert werden. Zur Aufarbeitung entfernt man hierbei überschüssiges wasserentziehendes Mittel unter vermindertem Druck und kristallisiert gegebenenfalls zur Reinigung um. Anstelle der Anthranilsäure kann auch das Carbonsäurehalogenid vorgelegt werden. Verwendet man Dicyclohexylcarbodiimid oder Thionylchlorid als wasserentziehendes Mittel, läßt sich die Cyclisierung mit der 1- bis 4fachen molaren Menge durchführen.

Zur Isolierung der 4H-3,1-Benzoxazinderivate der Formel I aus der Reaktionsmischung kann man diese mit Wasser, verdünntem Alkali oder verdünnter Säure zur Abtrennung von Nebenprodukten, wie nicht umgesetzter Anthranilsäure, Säurechlorid bzw. Basenhydrochlorid, behandeln, trocknen und dann einengen. Gegebenenfalls können die Endprodukte auch durch Umkristallisation oder Chromatographie gereinigt werden.

Die folgenden Beispiele erläutern die Herstellung der erfindungsgemäßen 4H-3,1-Benzoxazinderivate der Formel I. Gewichtsteile verhalten sich zu Volumenteilen wie Kilogramm zu Liter.

Beispiel 1

Herstellung von 2-(3'-Fluorphenyl)-5-chlor-4H-3,1-benzoxazin-4-on

a) 15,9 Teile 3-Fluorbenzoylchlorid und 10,1 Teile Triethylamin werden gleichzeitig über 2 Zuführungen bei 25 bis 30°C in eine Lösung von 17,1 Teilen 6-Chloranthranilsäure in 200 Teile Methylenchlorid eingeführt. Das Reaktionsgemisch wird 30 Minuten nachgerührt, einmal mit 1 n Salzsäure und mit Wasser extrahiert. Nach dem Trocknen und Einengen werden 29 Teile N-(3'-Fluorbenzoyl)-6-chloranthranilsäure vom Fp. 216 bis 219°C erhalten; Ausbeute: 98,6% d. Th.

b) 29 Teile N-(3'-Fluorbenzoyl)-6-chloranthranilsäure werden in 250 Teilen Essigsäureanhydrid zweieinhalb Stunden unter Rückfluß gerührt. Nach dem Einengen unter vermindertem Druck wird der Rückstand in Methylenchlorid aufgenommen, zweimal mit je 100 Volumenteilen 0,5 n Natronlauge und mit Wasser gewaschen. Anschließend wird die Lösung über neutrales Aluminiumoxid chromatographiert, wobei 23,6 Teile 2-(3'-Fluorphenyl)-5-chlor-4H-3,1-benzoxazin-4-on vom Fp. 123 bis 125°C erhalten werden; Ausbeute: 86,7% d. Th.

Beispiel 2

Herstellung von 2-(4'-Fluorphenyl)-5-chlor-4H-3,1-benzoxazin-4-on

a) 17,5 Teile 4-Fluorbenzoylchlorid und 14 Teile N,N-Dimethylcyclohexylamin werden gleichzeitig über 2 Zuführungen bei 10 bis 20°C zu einer Lösung von 17,1 Teilen 6-Chloranthranilsäure in 230 Teilen Essigester eingeführt und 20 Minuten bei 30°C nachgerührt. Dann wird das Reaktionsgemisch einmal mit 1 n Salzsäure und mit Wasser gewaschen, wobei ein farbloser Niederschlag ausfällt. Dieser wird in Essigester gelöst, die organische Phase wird nochmals mit 1 n Salzsäure und mit Wasser gewaschen, über Magnesiumsulfat getrocknet und eingeengt; hierbei werden 29 Teile N-(4'-Fluorbenzoyl)-6-chloranthranilsäure vom Fp. 228 bis 233°C erhalten.

b) 15,5 Teile Thionylchlorid werden bei Raumtemperatur zu einer Suspension von 29 Teilen N-(4'-Fluorbenzoyl)-6-chloranthranilsäure in 250 Teilen 1,2-Dichlorethan gegeben und dann eine Stunde unter Rückfluß gerührt. Anschließend wird die Reaktionsmischung eingeengt, in Methylenchlorid aufgenommen, einmal mit Wasser und zweimal mit je 100 Volumenteilen 0,5 n Natronlauge extrahiert. Nach dem Trocknen, Chromatographieren über neutralem Aluminiumoxid und Einengen werden 22,5 Teile 2-(4'-Fluorphenyl)-5-chlor-4H-3,1-benzoxazin-4-on vom Fp. 159 bis 162°C isoliert; Ausbeute: 82,7%.

Beispiel 3

Herstellung von 2-(3'-Trifluormethyl)-5-methyl-4H-3,1-benzoxazin-4-on

a) 15,4 Teile 3-Trifluormethylbenzoylfluorid werden unter Rühren bei 20 bis 25°C zu einer Mischung von 13,3 Teilen 6-Methylanthranilsäure und 16,3 Teilen Tri-n-butylamin in 250 Teilen 1,2-Dichlorpropan gegeben und 20 Minuten nachgerührt. Der ausgefallene Niederschlag wird abgesaugt und mit 1 n Salzsäure und mit Wasser gewaschen; ebenso wird das organische Filtrat gewaschen, getrocknet und eingeengt. Man erhält dabei 27 Teile n-(3'-Trifluormethylbenzoyl)-6-methylanthranilsäure vom Fp. 198 bis 204°C.

b) 27 Teile N-3'-Trifluormethylbenzoyl-6-methylanthranilsäure werden in 200 Teilen Acetanhydrid zwei Stunden unter Rückfluß gerührt. Nach dem Einengen unter vermindertem Druck wird der Rückstand in Methylenchlorid aufgenommen, zweimal mit je 100 Volumenteilen 0,5 n Natronlauge und mit Wasser gewaschen. Anschließend wird die Lösung über neutrales Aluminiumoxid chromatographiert, wobei 23 Teile 2-(3'-Trifluormethyl)-5-methyl-4H-3,1-benzoxazin-4-on erhalten werden vom Fp. 120 bis 123°C; Ausbeute: 89,6%.

Nach dem entsprechenden Verfahren können beispielsweise folgende 4H-3,1-Benzoxazinderivate der Formel I hergestellt werden:

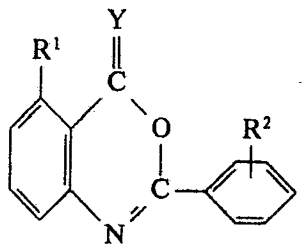

| Nr. | $R^1$ | $R^2$ | Y | Fp [°C] |
|---|---|---|---|---|
| 4 | Cl | m-Cl | O | 170—173 |
| 5 | Cl | m-F | S | |
| 6 | Cl | p-Cl | O | 208—210 |
| 7 | Cl | p-F | S | |
| 8 | Cl | p-J | O | 188—191 |
| 9 | Cl | m-Br | O | 206—208 |
| 10 | Cl | p-Br | O | 202—206 |
| 11 | F | m-F | O | 135—138 |
| 12 | F | m-F | S | |
| 13 | F | m-Cl | O | 182—184 |
| 14 | F | p-Cl | O | 199—201 |
| 15 | F | p-F | O | 192—194 |
| 16 | F | p-F | S | |
| 17 | $CH_3$ | m-$CF_3$ | S | |
| 18 | $CH_3$ | m-O—$CF_3$ | O | 90— 93 |
| 19 | $CH_3$ | m-O—$CF_2Cl$ | O | 83— 86 |
| 20 | $CH_3$ | m-$OCF_2Cl$ | S | |
| 21 | $CH_3$ | m-F | O | 125—128 |
| 22 | $NO_2$ | m-O—$CF_3$ | O | 103—105 |
| 23 | $NO_2$ | m-O—$CF_2Cl$ | O | 123—126 |

Die erfindungsgemäßen Substanzen können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Diselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tatrachlorkohlenstoff,

Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächennaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen. Laurylalkoholpolyglykoletheracetal, Sorbitester, Lignin, Sulfitablaugen und Methylcellulose in Betracht.

Pulver-, Streu- und Stäubemitel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalennmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung gemäß Beispiel 1 mit 10 Gewichtsteilen N-Methyl-$\alpha$-pyrrolidon und erhält eine Lösung, die zur Anwendung in Form kleinster Tropfen geeignet ist.

II. 10 Gewichtsteile der Verbindung gemäß Beispiel 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile der Verbindung Nr. 4 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile der Verbindung Nr. 11 werden in einer Mischung gelöst, die aus 25 Gewichtsteilen Cyclohexanol, 65 Gewichtsteilen einer Mineralölfraktion vom Siedepunkt 210 bis 280° C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile der Verbindung Nr. 6 werden in 3 Gewichtsteilen des Natriumsalzes der Diisobutylnaphthalin-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile der Verbindung Nr. 13 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile der Verbindung Nr. 14 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 15 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Der Einfluß verschiedener Vertreter der 4H-3,1-Benzoxazinderivate der Formel I auf das Wachstum von unerwünschten Pflanzen wird durch Gewächshausversuche gezeigt:

Als Kulturgefäße dienten Plastikblumentöpfe mit 300 $cm^3$ Inhalt und lehmigem Sand mit etwa 1,5% Humus als Substrat. Bei Reis wurden für die Nachauflaufbehandlung höhere Torfanteile zugemischt, um ein besseres Wachstum zu gewährleisten. Die Samen der Testpflanzen wurden nach Arten getrennt flach eingesät.

Bei der Vorauflaufbehandlung wurden die Wirkstoffe auf die Erdoberfläche aufgebracht. Sie wurden hierzu in Wasser als Verteilungsmittel suspendiert oder emulgiert und mittels fein verteilender Düse gespritzt. Bei dieser Applikationsmethode betrug die Aufwandmenge 3,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Die Abdeckung bewirkte ein gleichmäßiges Keimen der Testpflanzen, sofern dies nicht durch die Wirkstoffe beeinträchtigt wurde.

Zum Zwecke der Nachauflaufbehandlung zog man die Testpflanzen je nach Wuchsform erst bis zu einer Wuchshöhe von 3 bis 10 cm an und behandelte sie danach. Zur Nachauflaufbehandlung wurden entweder direkt gesäte und in den gleichen Gefäßen aufgewachsene Pflanzen ausgewählt, oder sie wurden erst als Keimpflanzen getrennt angezogen und einige Tage vor der Behandlung in die Versuchsgefäße verpflanzt. Die Aufwandmenge für die Nachauflaufbehandlung variierte je nach Wirkstoff. Sie betrug im Einzelfall 0,5 oder 1,0 kg Wirkstoff/ha. Als Vergleichsmittel wurde 2-Phenyl-4H-3,1-benzoxazin-4-on (BE-PS 648 259) mit 1,0 kg/ha verwendet. Eine Abdeckung unterblieb bei der Nachauflaufbehandlung. Die Versuchsgefäße wurden im Gewächshaus aufgestellt, wobei für wärmeliebende Arten wärmere Bereiche (20 bis 35°C) und für solche gemäßigter Klimate 10 bis 25°C bevorzugt wurden. Die Versuchsperiode erstreckte sich über 2 bis 4 Wochen. Während dieser Zeit wurden die Pflanzen gepflegt, und ihre Reaktion auf die einzelnen Behandlungen ausgewertet. Bewertet wurde nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Bei den Versuchen wurden folgende Pflanzen getestet: Arachys hypogaea (Erdnuß), Avena sativa (Hafer), Cassia tora, Centaurea cyanus (Kornblume) Chenopodium spp. (Gänsefuß), Chrysanthemum spp. (Saatwucherblumenarten), Datura stramonium (gemeiner Stechapfel), Desmodium tortuosum, Euphorbia geniculata (Südamerik. Wolfsmilchart), Galium aparine (Klettenlabkraut), Gossypium hirsutum (Baumwolle), Ludwigia spp. (Heusenkrautart), Mercurialis annua (Einjähriges Bingelkraut), Sinapis alba (Weißer Senf), Solanum nigrum (Schwarzer Nachtschatten), Oryza sativa (Reis), Triticum aestivum (Weizen) und Zea mays (Mais).

Bei der Prüfung der herbiziden Wirkung bei Vorauflaufanwendung im Gewächshaus bei Aufwandmengen von 3,0 kg Wirkstoff/ha zeigen die neuen Verbindungen Nr. 11, 22, 23 eine gute herbizide Aktivität bei guter Verträglichkeit für Hafer als Vertreter für Getreidearten.

Bei der Prüfung auf selektive herbizide Wirkung bei Nachauflaufanwendung im Gewächshaus zeigen die Wirkstoffe Nr. 22 bei 1,0 kg/ha und Nr. 23 bei 0,5 kg/ha eine bessere herbizide Wirkung und eine bessere Kulturpflanzenverträglichkeit für Baumwolle als der bekannte Wirkstoff A bei 1,0 kg Wirkstoff/ha. Die Wirkstoffe Nr. 1 und Nr. 11 zeigen bei der selektiven Unkrautbekämpfung bei Nachauflaufanwendung im Gewächshaus ebenfalls bei 1,0 bzw. 0,5 kg Wirkstoff/ha eine sehr gute herbizide und selektive Wirkung.

Bei der Prüfung ebenfalls auf selektive herbizide Wirkung bei Nachauflaufanwendung im Gewächshaus zeigen die Wirkstoffe Nr. 18 und 19 bei einer Aufwandmenge von 1,0 kg/ha eine sehr gute Verträglichkeit bei Weizen und eine sehr gute Wirkung bei Solanum nigrum als Vertreter für Unkräuter.

Bei der Prüfung der Wirkstoffe Nr. 2 und Nr. 15 bei 3,0 kg/ha bei Nachauflaufanwendung im Gewächshaus entfalteten diese eine beachtliche herbizide Aktivität gegen breitblättrige Unkräuter, besonders Calium aparine, bei sehr guter Verträglichkeit bei Getreide, beispielsweise Hafer.

Sind gewisse Kulturpflanzen bei Blattbehandlung gbegenüber den Wirkstoffen etwa empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die Blätter darunter wachsender unerwünschter Pflanzen oder die unbedeckte Bodenfläche gelangen (post-directed, lay-by).

In Anbetracht der guten Verträglichkeit und der Vielseitigkeit der Applikationsmethoden können die erfindungsgemäßen Wirkstoffe und diese enthaltende herbizide Mittel noch in einer weiteren großen Zahl von Kulturpflanzen zur Beseitigung unerwünschten Pflanzenwuchses eingesetzt werden. Die Aufwandmengen können dabei zwischen 0,1 und 15 kg Wirkstoff/ha und mehr, vorzugsweise zwischen 0,5 und 3,5 kg Wirkstoff/ha schwanken. Sie richten sich nach dem jeweiligen Bekämpfungsziel, dem Stadium der unerwünschten Pflanzen und der Jahreszeit.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. naprobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor-Färbedistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea cane phora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |

(Fortsetzung)

| Botanischer Name | Deutscher Name |
|---|---|
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactuca sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Urdbohne |
| Phaseolus vulgaris | Buschbohnen |
| Pennisetum glaucum | Perl- oder Rohrkolbenhirse |
| Petroselinim crispum spp. tuberosum | Wurzelpetersilie |
| Picea abies | Rotfichte |
| Abies alba | Weißtanne |
| Pinus spp. | Kiefer |

(Fortsetzung)

| Botanischer Name | Deutscher Name |
|---|---|
| Pisum sativum | Gartenerbse |
| Prunus avium | Süßkirsche |
| Prunus domestica | Pflaume |
| Prunus dulcis | Mandelbaum |
| Prunus persica | Pfirsich |
| Prunus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) Mohrenhirse | |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen 4H-3,1-Benzoxazin-4-one sowohl unter sich als auch mit zahlreichen Vertretern anderer herbizider oder wachstumsregulierender Wirkstoffgruppen gemischt und gemeinsam ausgebracht werden. Beispielsweie kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäure, Triazine, Amide, Harnstoffe, Diphenylether, Triazinone, Uracile, Benzofuranderivate, Cyclohexan-1,3-dionderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt:

5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon

5-Methylamino-4-chlor-2-(3-trifluormethylphenyl)-3(2H)-pyridazinon
5-Methylamino-4-chlor-2-(3-α,α,β,β-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3-trifluormethylphenyl)-3(2H)-pyridazinon
5-Methoxy-4-chlor-2-(3-trifluormethylphenyl)-3(2H)-pyridazinon
5-Amino-4-brom-2-(3-methylphenyl)-3(2H)-pyridazinon

4,5-Dimethoxy-2-(3-α,α,β-trifluor-β-bromethoxyphenyl)-3(2H)-pyridazinon
3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl)2,1,3-benzothiadiazin-4(3H)-on-2,2-dioxid
1-Methoxymethyl-8-chlor-3-(1-methylethyl)2,1,3-benzothiadiazin-4(3H)-on-2,2-dioxid
1-Methoxymethyl-8-fluor-3-(1-methylethyl)2,1,3-benzothiadiazin-4(3H)-on-2,2-dioxid
1-Cyan-8-chlor-3-(1-methylethyl)-2,1,3-benzothiadiazin-4(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Cyan-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-pyridino-[3,2-e]2,1,3-thiadiazin-(4)-on-2,2-dioxid

N-(1-Ethylpropyl)-2,6-dinitro-3,4-dimethylanilin
N-(1-Methylethyl)-N-ethyl-2,6-dinitro-4-trifluormethyl-anilin
N-n-Propyl-N-β-chlorethyl-2,6-dinitro-4-trifluormethyl-anilin
N-n-Propyl-N-cyclopropylmethyl-2,6-dinitro-4-trifluor-methyl-anilin
N-Bis-(n-propyl)-2,6-dinitro-3-amino-4-trifluormethylanilin
N-Bis-(n-propyl)-2,6-dinitro-4-methyl-anilin
N-Bis-(n-propyl)-2,6-dinitro-4-methylsulfonyl-anilin
N-Bis-(n-propyl)-2,6-dinitro-4-aminosulfonyl-anilin
Bis-(β-Chlorethyl)-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert-butyl-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3′-N-Isopropyl-carbamoyloxy-propionanilid
Ethyl-N-(3-(N′-phenylcarbamoyloxy)-phenyl)-carbamat
Methyl-N-(3-(N′-methyl-N′-phenylcarbamoyloxy)-phenyl)-carbamat
Isopropyl-N-(3-(N′-ethyl-N′-phenylcarbamoyloxy)-phenyl)-carbamat
Methyl-N-(3-(N′-methyl-3-methylphenylcarbamoyloxy)-phenyl)-carbamat
Methyl-N-(3-(N′-4′-fluorphenylcarbamoyloxy)-phenyl)-carbamat
Methyl-N-(3-(N′-3-chlor-4-fluorphenylcarbamoyloxy)-phenyl)-carbamat
Methyl-N-dichlorfluormethylsulfenyl-(3-(N′-dichlorfluormethylsulfenyl-N′-phenylcarbamoyl-oxy)-phenyl)-carbamat
Methyl-N-dichlorfluormethylsulfenyl-(3-(N′-dichlorfluormethylsulfenyl-N′-3-methylphenylcarbamoyl-oxy)-phenyl)-carbamat
Ethyl-N-(3-N′-3-chlor-4-fluorphenylcarbamoyloxy)-phenyl)-carbamat
Ethyl-N-(3-N′-3,4-difluorphenylcarbamoyloxy)-phenyl)-carbamat

Methyl-N-(3-(N'-3,4-difluorphenylcarbamoyloxy)-phenyl)-carbamat
N-3-(4-Fluorphenoxycarbonylamino)-phenyl-carbaminsäure-methylester
N-3-(2-Methylphenoxycarbonylamino)-phenyl-carbaminsäure-ethylester
N-3-(4-Fluorphenoxycarbonylamino)-phenyl-thiolcarbaminsäure-methylester
N-3-(2,4,5-Trimethylphenoxycarbonylamino)-phenyl-thiocarbaminsäure-methylester
N-3-(Phenoxycarbonylamino)-phenyl-thiolcarbaminsäure-methylester
N,N-Diethyl-thiolcarbaminsäure-p-chlorbenzylester
N,N-Di-n-propyl-thiolcarbaminsäure-ester
N,N-Di-n-propyl-thiolcarbaminsäurepropylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.-butyl-thiolcarbaminsäure-ethylester
N,N-Di-sec.-butyl-thiolcarbaminsäure-benzylester
N-Ethyl-N-cyclohexyl-thiolcarbaminsäure-ethylester
N-Ethyl-N-bicyclo[2.2.1]heptyl-thiocarbaminsäureethylester

S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-(3-methyl-hexahydro-1-H-azepin-1)-carbothiolat
S-Benzyl-(2,3-dimethylhexahydro-1-H-azepin-1)-carbothiolat
S-Ethyl-(3-methylhexahydro-1-H-azepin-1)-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α,β,β-Tetrafluorpropionsäure-Natriumsalz
α-Methyl-α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure

2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyl-tetrachlor-thioterephthalat
Dimethyl-2,3,5,6-tetrachlor-terephthalat
Di-natrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäure-Natriumsalz
2-[4-(3',5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäure-methylester
2-(N-Benzoyl-3-chlor-4-fluorphenylamino)-propionsäureisopropylester
4-(4'-Trifluormethyl-phenoxy)-penten-2-carbonsäureethylester

2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-2-methoxypropyl-2-amino-1,3,5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert-butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisisoethylamino-1,3,5-triazin
2-Methoxy-4,6-bisethylpropylamino-1,3,5-triazin
4-Amino-6-tert.-butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.-butyl-3-methylthio-4,5-dihydro-1,2,4-triazin-5-on
1-Methyl-3-cyclohexyl-6-dimethylamino-1,3,5-triazin-2,4-dion

3-tert.-Butyl-5-chlor-6-methyluracil
3-tert.-Butyl-5-brom-6-methyluracil
3-Isopropyl-5-brom-6-methyluracil
3-sec.-Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6-trimethylenuracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl)-2-[1',2',4'-triazolyl-(1')]ethan (Salze)
1-(4-Chlorphenoxy)-3,3-dimethyl-1-(1H-1,2,4-triazol-1-yl)-butan-2-on
N,N-Diallylchloracetamid
N-Isopropyl-2-chloracetanilid
N-(Butin-1-yl-3)-2-chloracetanilid

2-Methyl-6-ethyl-N-(Propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl-methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-isobutoxymethyl-2-chloracetanilid
2,6-Diethyl-N-methoxymethyl-2-chloracetanilid
2,6-Diethyl-N-(n-butoxymethyl)-2-chloracetanilid
2,6-Diethyl-N-ethoxycarbonylmethyl-2-chloracetanilid
2,3,6-Trimethyl-N-(pyrazol-1-yl-methyl)-2-chloracetanilid
2,3-Dimethyl-N-isopropyl-2-chloracetanilid
2,6-Diethyl-N-(2-n-propoxyethyl)-2-chloracetanilid

2-(2-Methyl-4-chlorphenoxy)-N-methoxy-acetamid
2-($\alpha$-Naphthoxy)-N,N-diethylpropionamid
2,2-Diphenyl-N,N-dimethylacetamid
N-Benzyl-N-isopropyl-trimethylacetamid
$\alpha$-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyl-trifluormethansulfonanilid
5-Acetamido-4-methyl-trifluormethansulfonanilid
N-4-Methyl-5-(trifluormethyl)-sulfonylamino-phenylacetamid
2-Propionyl-amino-4-methyl-5-chlor-thiazol

O-(Methylsulfonyl)-glykolsäure-N-ethoxymethyl-2,6-dimethylanilid
O-(Methylaminosulfonyl)-glykolsäure-N-isopropyl-anilid
O-(i-Propylaminosulfonyl)-glykolsäure-N-butin-1-yl-3-anilid
O-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-O-2,4-dinitrophenylbenzaldoxim (Salze)
3,5-Dibrom-4-hydroxy-O-2-cyan-4-nitrophenylbenzaldoxim (Salze)
Pentachlorphenol-Natriumsalz
2,5-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether

2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2-Chlor-4-trifluormethylphenyl-3'-ethoxycarbonyl-4'-nitrophenylether
2-Chlor-4-trifluormethylphenyl-3'-(2-fluorethoxy)-4'-nitro-phenylether
2-Chlor-4-trifluormethylphenyl-3'-ethoxycarbonyl-methylthio-4'-nitrophenylether
2-Chlor-4-trifluormethyl-3'-methoxycarbonyl-4'-nitrophenylether
2,4,6-Trichlorphenyl-3'-ethoxycarbonyl-methylthio-4'-nitro-phenylether
2,4-Dichlorphenyl-3'-methoxycarbonyl-4'-nitro-phenylether
2,4-Dichlorphenyl-3'-carboxy-4'-nitrophenylether
2-(3,4-Dichlorphenyl)-4-methyl-1,2,4-oxadiazolidin-3,5-dion
2-(3-tert.-Butylcarbamoyloxy-phenyl)-4-methyl-1,2,4-oxadiazolidin-3,5-dion
2-(3-i-Propylcarbamoyloxy-phenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$,$O^{8,11}$]-dodeca-3,9-dien
2-Ethoxy-2,3-dihydro-3,3-dimethyl-5-benzofuranyl-methan-sulfonat
2-Ethoxy-2,3-dihydro-3,3-dimethyl-5-benzofuranyl-dimethyl-aminosulfat
2-Ethoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl-allylbernnsteinsäureimid
2-Methyl-4,6-dinitrophenol (Salze, Ester)
2-sec.-Butyl-4,6-dinitrophenol (Salze, Ester)
2-sec.-Butyl-4,6-dinitrophenol-acetat
2-tert.-Butyl-4,6-dinitrophenol-acetat
2-tert.-Butyl-4,6-dinitrophenol (Salze)
2-tert.-Butyl-5-methyl-4,6-dinitrophenol (Salze)
2-tert.-Butyl-5-methyl-4,6-dinitrophenol-acetat

2-sec.-Amyl-4,6-dinitrophenol (Salze, Ester)
1-($\alpha$,$\alpha$-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3.dimethyl-harnstoff
1-(3,4-Dichlorphenyl)-1-benzoyl-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-$\alpha$,$\alpha$,$\beta$,$\beta$-Tetrafluorethoxyphenyl)-3,3-dimethyl-harnstoff

1-(3-tert.-Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4-(4'-Chlorphenoxy)-phenyl]-3,3-dimethyl-harnstoff
1-[4-(4'-Methoxyphenoxy)-phenyl]-3,3-dimethyl-harnstoff

1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-3-methoxy-harnstoff
1-(3-tert.-Butylphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
1-(4-Benzyloxyphenyl)-3-methyl-3-methoxy-harnstoff
Imidazolidin-2-on-1-carbonsäure-isobutylamid
1,2-Dimethyl-3,5-diphenylpyrazolium-methylsulfat
1,2-4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[(4-methylphenyl)-sulfonyloxy]-pyrazol
1-Acetyl-3-anilino-4-methoxycarbonyl-5-methyl-pyrazol
3-Anilino-4-methoxycarbonyl-5-methyl-pyrazol
3-tert.-Butylamino-4-methoxycarbonyl-5-methyl-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di-methylsulfat
1,1'-Di-(3,5-dimethylmorpholin-carbonylmethyl)-4,4'-dipyridylium-dichlorid
1,1'-Ethylen-2,2'-dipyridylium-dibromid

2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)

3,5,6-Trichlor-2-pyridinyl-oxyessigsäure (Salze, Ester, Amide)

α-Naphthoxyessigsäuremethylester
2-[4-(5'-Brompyridyl-2-oxy)-phenoxy]-propionsäureethylester
2-[4-(5'-Iodpyridyl-2-oxy)-phenoxy]-propionsäureethylester
2-[4-(5'-Iodpyridyl-2-oxy)-phenoxy]-propionsäure-n-butylester
2-(2-Methylphenoxy)-propionsäure (Salze, Ester, Amide)
2-(4-Chlorphenoxy]-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
2-[4-(4'-Chlorphenoxymethyl)-phenoxy]-propionsäuremethylester
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)

Gibellerinsäure (Salze)
Dinatrium-methylarsonat
Mononatriumsalz der Methylarsonsäure
N-Phosphon-methyl-glycin (Salze)
N,N-Bis-(phosphormethyl)-glycin (Salze)
2-Chlorethanphosphonsäure-2-chlorethylester
Ammonium-ethyl-carbamoyl-phosphonat

Di-n-butyl-1-n-butylamino-cyclohexyl-phosphonat
Trithiobutylphosphit
O,O-Diisopropyl-5-(2-benzosulfonylamino-ethyl)-phosphordithionat
2,3-Dihydro-5,6-dimethyl-1,4-dithiin-1,1,4,4-tetraoxid
5-tert.-Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
2-[1-(2,5-Dimethylphenyl)-ethylsulfonyl]-pyridin-N-oxid
2-(3'-Trifluormethyl-phenyl)-4H-3,1-benzoxazin-4-on
2-(2-Thienyl)-4H-3,1-benzoxazin-4-on
Natriumchlorat
Ammoniumrhodanid
Calciumcyanamid

2-Chlor-4-trifluormethyl-3-ethoxycarbonyl-4'-nitrophenylether

1-(4-Benzyloxyphenyl)-3-methyl-3-methoxyharnstoff
2-[1-(2,5-Dimethylphenyl)-ethylsulfonyl]-pyridin-N-oxid
1-Acetyl-3-anilino-4-methoxycarbonyl-5-methylpyrazol
3-Anilino-4-methoxycarbonyl-5-methylpyrazol
3-tert.-Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Iodpyridyl-2-oxy)-phenoxy]-propionsäureethylester
2-[4-(5'-Iodpyridyl-2-oxy)-phenoxy]-propionsäure-n-butylester
2-Chlor-4-trifluormethylphenyl-3'-(2-fluorethoxy)-4'-nitro-phenylether
2-Chlor-4-trifluormethylphenyl-3-(ethoxycarbonyl)-methylthio-4-nitrophenylether
2,4,6-Trichlorpenyl-3'-(ethoxycarbonyl)-methylthio-4-nitrophenylether
2-[1-(N-ethoxamino)-butyliden]-5-(2-ethylthiopropyl)-3-hydroxy-cyclohexen-(2)-on-1) (Salze)
2-[1-(N-ethoxamino)-butyliden]-5-(2-phenylthiopropyl)-3-hydroxy-cyclohexen-(2)-on-(1) (Salze)

4-[4-(4'-Trifluormethyl)-phenoxy]-penten-2-carbonsäureethylester
2-Chlor-4-trifluormethylphenyl-3'-methoxycarbonyl-4'-nitrophenylether
2,4-Dichlorphenyl-3'-carboxy-4'-nitrophenylether (Salze)
4,5-Dimethoxy-2-(3-$\alpha,\alpha,\beta$-trifluor-$\beta$-bromethoxyphenyl)-3-(2H)-pyridazinon

2,4-Dichlorphenyl-3'-ethoxy-ethoxy-ethoxy-4'-nitrophenyl-ether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzolsulfonamid
1-(3-Chlor-4-ethoxyphenyl)-3,3-dimethylharnstoff
2-Methyl-4-chlorphenoxy-thioessigsäureethylester
2-Chlor-3,5-dijodid-4-acetoxy-pyridin
1-(4-[2-(4-Methylphenyl)-ethoxy]-phenyl)-3-methyl-3-methoxyharnstoff
2,6-Dimethyl-N-(pyrazol-1-yl-methylenoxymethyl)-2-chloracetanilid
2-Methyl-6-ethyl-N-(pyrazol-1-yl-methylenoxymethyl)-2-chloracetanilid
1-($\alpha$-2,4-Dichlorphenoxypropionsäure)-3-(O-methylcarbamoyl)-anilid
1-($\alpha$-2-Brom-4-chlorphenoxypropionsäure)-3-(O-methylcarbamoyl)-anilid
2-Methyl-6-ethyl-N-(pyrazol-1-yl-ethylenoxymethyl)-2-chloracetanilid

Methyl-N-dichlorfluormethylsulfenyl-(3-(N'-dichlorfluormethylsulfenyl-N'-phenylcarbamoyl-oxy)-phenyl)-carbamat
Methyl-N-dichlorfluormethylsulfenyl-(3-(N'-dichlorfluormethylsulfenyl-N'-3-methylphenylcarbamoyl-oxy)-phenyl)-carbamat

N-(Pyrazol-1-yl-methyl)-pyrazol-1-yl-essigsäure-2,6-dimethylanilid
N-(Pyrazol-1-yl-methyl)-1,2,4-triazol-1-yl-essigsäure-2,6-dimethylanilid

2-(3'-Trifluormethylphenyl)-4H-3,1-benzoxazin-4-on
2-(2-Thienyl)-4H-3,1-benzoxazin-4-on

3-[1-(N-Ethoxyamino)-propyliden]-6-ethyl-3,4-dihydro-2H-pyran-2,4-dion
3-[1-(N-Allyloxyamino)-propyliden]-6-ethyl-3,4-dihydro-2H-pyran-2,4-dion
2-[1-(N-Allylooxyamino)-propyliden]-5,5-dimethylcyclohexan-1,3-dion (Salze)
2-[1-(N-Allyloxyamino)-butyliden]-5,5-dimethylcyclohexan-1,3-dion (Salze)
2-[1-(N-Allyloxyamino)-butyliden]-5,5-dimethyl-4-methoxycarbonyl-cyclohexan-1,3-dion (Salze).

Außerdem ist es nützlich, die erfindungsgemäßen Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden.

Zur Aktivierung der herbiziden Wirkung können auch Netz- und Haftmittel sowie nicht-phytotoxische Öle und Ölkonzentrate zugesetzt werden.

**Patentansprüche für die Vertragsstaaten: BE, CH, DE, FR, GB, IT, LI, LU, NL, SE**

1. 4H-3,1-Benzoxazinderivate der Formel I

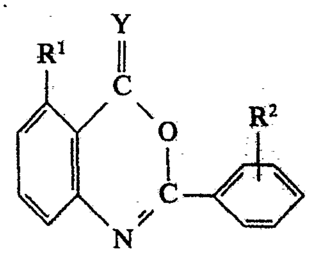

(I)

in der

Y Sauerstoff oder Schwefel und
$R^1$ Halogen bedeutet, wenn $R^2$ Halogen in m- oder p-Stellung ist, oder
$R^1$ Methyl bedeutet, wenn $R^2$ Fluor, Trifluormethyl, Trifluormethoxy oder Chlordifluormethoxy in m-Stellung ist, oder
$R^1$ Nitro bedeutet, wenn $R^2$ Trifluormethoxy oder Chlordifluormethoxy in m-Stellung ist.

2. 4H-3,1-Benzoxazinderivate der Formel I gemäß Anspruch 1, dadurch gekennzeichnet, daß Y Sauerstoff, $R^1$ Halogen und $R^2$ Halogen in m- oder p-Stellung bedeuten.
3. 2-(3'-Fluorphenyl)-5-chlor-4H-3,1-benzoxazin-4-on.
4. 2-(3'-Fluorphenyl)-5-chlor-4H-3,1-benzoxazin-4-on.
5. Verfahren zur Herstellung von 4H-3,1-Benzoxazinderivaten der Formel I gemäß Anspruch 1, dadurch gekennzeichnet, daß man eine substituierte Anthranilsäure der Formel II

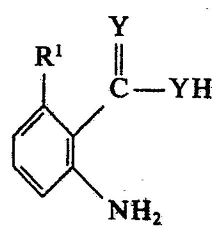

(II)

in der
$R^1$ und Y die im Anspruch 1 genannten Bedeutungen haben, mit mindestens dem zweifachen molaren Überschuß eines Carbonsäurehalogenids der Formel III

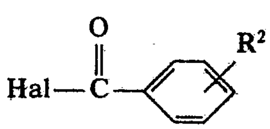

(III)

in der
$R^2$ die im Anspruch 1 genannten Bedeutungen hat und Hal für Halogen steht, in einem aromatischen tertiären Amin als Lösungsmittel bei einer Temperatur im Bereich zwischen 10 und 60° C umsetzt.

6. Verfahren zur Herstellung von 4H-3,1-Benzoxazinderivaten der Formel I gemäß Anspruch 1, dadurch gekennzeichnet, daß man eine substituierte Anthranilsäure der Formel II

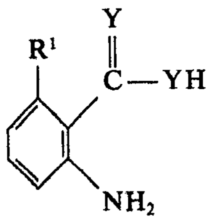

(II)

in der
$R^1$ und Y die im Anspruch 1 genannten Bedeutungen haben, oder ein Alkalimetall- oder Erdalkalimetallsalz dieser Anthranilsäure mit ungefähr stöchiometrischen Mengen eines Carbonsäurehalogenids der Formel III

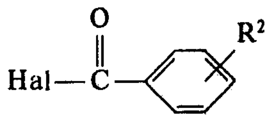

(III)

in der
$R^2$ die im Anspruch 1 genannten Bedeutungen hat und Hal für Halogen, steht in einem inerten organischen Lösungsmittel oder in Wasser und gegebenenfalls in Anwesenheit eines Säureakzeptors bei einer Temperatur im Bereich von 0 bis 60° C zu einem Carbonamid der Formel IV

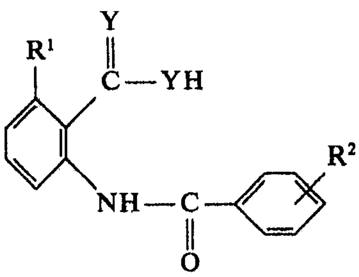

(IV)

in der
$R^1$, $R^2$ und Y die im Anspruch 1 genannten Bedeutungen haben, umsetzt und dieses dann in Gegenwart eines wasserentziehenden Mittels bei einer Temperatur im Bereich zwischen 30 und 150° C cyclisiert.

7. Herbizid, enthaltend ein 4H-3,1-Benzoxazinderivat der Formel I gemäß Anspruch 1.

8. Herbizid, enthaltend inerte Zusatzstoffe und ein 4H-3,1-Benzoxazinderivat der Formel I gemäß Anspruch 1.

9. Verfahren zur Bekämpfung unerwünschten Pflanzenwuchses, dadurch gekennzeichnet, daß man die Pflanzen und/oder ihren Standort mit einer herbizid wirksamen Menge eines 4H-3,1-Benzoxazinderivats der Formel I gemäß Anspruch 1 behandelt.

**Patentansprüche für den Vertragsstaat: AT**

1. Herbizid, enthaltend inerte Zusatzstoffe und ein 4H-3,1-Benzoxazinderivat der Formel I

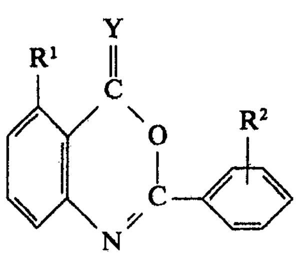

(I)

in der

Y Sauerstoff oder Schwefel und
$R^1$ Halogen bedeutet, wenn $R^2$ Halogen in m- oder p-Stellung ist, oder
$R^1$ Methyl bedeutet, wenn $R^2$ Fluor, Trifluormethyl, Trifluormethoxy oder Chlordifluormethoxy in m-Stellung ist, oder
$R^1$ Nitro bedeutet, wenn $R^2$ Trifluormethoxy oder Chlordifluormethoxy in m-Stellung ist,

als Wirkstoff.

2. Herbizid, enthaltend inerte Zusatzstoffe und ein 4H-3,1-Benzoxazinderivat der Formel I gemäß Anspruch 1, in der Y Sauerstoff, $R^1$ Halogen und $R^2$ Halogen in m- oder p-Stellung bedeuten, als Wirkstoff.

3. Herbizid, enthaltend inerte Zusatzstoffe und 2-(3'-Fluorphenyl)-5-chlor-4H-3,1-benzoxazin-4-on als Wirkstoff.

4. Herbizid, enthaltend inerte Zusatzstoffe und 2-(3'-Fluorphenyl)-5-fluor-4H-3,1-benzoxazin-4-on als Wirkstoff.

5. Verfahren zur Herstellung von 4H-3,1-Benzoxazinderivaten der Formel I gemäß Anspruch 1, dadurch gekennzeichnet, daß man eine substituierte Anthranilsäure der Formel II

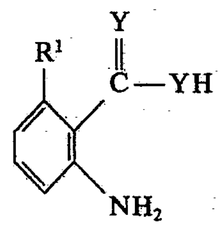

(II)

in der
$R^1$ und Y die im Anspruch 1 genannten Bedeutungen haben, mit mindestens dem zweifachen molaren Überschuß eines Carbonsäurehalogenids der Formel III

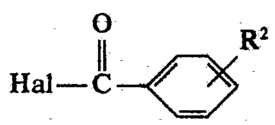

(III)

in der
$R^2$ die im Anspruch 1 genannten Bedeutungen hat und Hal für Halogen steht, in einem aromatischen tertiären Amin als Lösungsmittel bei einer Temperatur im Bereich zwischen 10 und 60°C umsetzt.

6. Verfahren zur Herstellung von 4H-3,1-Benzoxazinderivaten der Formel I gemäß Anspruch 1, dadurch gekennzeichnet, daß man eine substituierte Anthranilsäure der Formel II

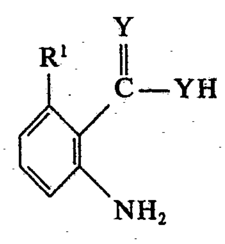

(II)

in der
$R^1$ und Y die im Anspruch 1 genannten Bedeutungen haben, oder ein Alkalimetall- oder Erdalkalimetallsalz dieser Anthranilsäure mit ungefähr stöchiometrischen Mengen eines Carbonsäurehalogenids der Formel III

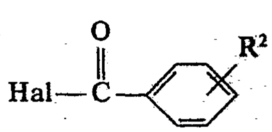

(III)

in der
$R^2$ die im Anspruch 1 genannten Bedeutungen hat und Hal für Halogen, steht in einem inerten organischen Lösungsmittel oder in Wasser und gegebenenfalls in Anwesenheit eines Säureakzeptors

bei einer Temperatur im Bereich von 0 bis 60° C zu einem Carbonamid der Formel IV

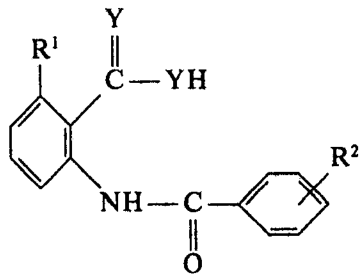

(IV)

in der
$R^1$, $R^2$ und Y die im Anspruch 1 genannten Bedeutungen haben, umsetzt und dieses dann in Gegenwart eines wasserentziehenden Mittels bei einer Temperatur im Bereich zwischen 30 und 150° C cyclisiert.

**Claims for the Contracting states: BE, CH, LI, DE, FR, GB, IT, LU, NL, SE**

1. A 4H-3,1-benzoxazine derivate of the formula I

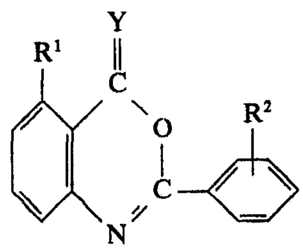

(I)

where Y is oxygen or sulfur, and $R^1$ is halogen if $R^2$ is halogen in the m- or p-position, or $R^1$ is methyl if $R^2$ is fluorine, trifluoromethyl, trifluoromethoxy or chlorodifluoromethoxy in the m-position, or $R^1$ is nitro if $R^2$ is trifluoromethoxy or chlorodifluoromethoxy in the m-position.

2. A 4H-3,1-benzoxazine derivative of the formula I as claimed in claim 1, where Y is oxygen, $R^1$ is halogen and $R^2$ is halogen in the m- or p-position.

3. 2-(3′-Fluorphenyl)-5-chloro-4H-3,1-benzoxazin-4-one.

4. 2-(3′-Fluorphenyl)-5-fluoro-4H-3,1-benzoxazin-4-one.

5. A process for the manufacture of a 4H-3,1-benzoxazine derivative of the formula I as claimed in claim 1, wherein a substituted anthranilic acid of the formula II

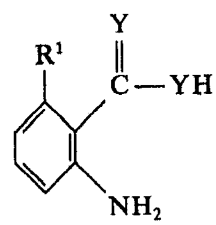

(II)

where $R^1$ and Y have the meanings given in claim 1, is reacted with not less than a two-fold molar excess of a carboxylic acid halide of the formula III

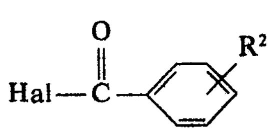

(III)

where $R^2$ has the meanings given in claim 1 and Hal is halogen, in an aromatic tertiary amine as the solvent, at from 10 to 60° C.

6. A process for the manufacture of a 4H-3,1-benzoxazine derivative of the formula I as claimed in claim 1, wherein a substituted anthranilic acid of the formula II

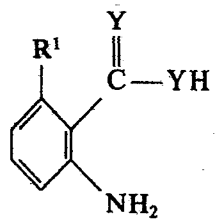

(II)

where $R^1$ and Y have the meanings given in claim 1, or an alkali metal salt or alkaline earth metal salt of this anthranilic acid, is reacted with about the stoichiometric amount of a carboxylic acid halide of the formula III

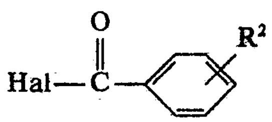

(III)

where $R^2$ has the meanings given in claim 1 and Hal is halogen, in an inert organic solvent or in water, in the presence or absence of an acid acceptor, at from 0 to 60° C, to give a carboxamide of the formula IV

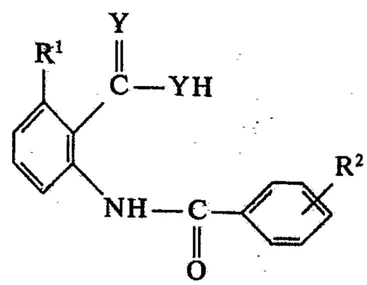

(IV)

where $R^1$, $R^2$ and Y have the meanings given in claim 1, and then the carboxamide is cyclized in the presence of a dehydrating agent at from 30 to 150° C.

7. A herbicide containing a 4H-3,1-benzoxazine derivative of the formula I as claimed in claim 1.

8. A herbicide containing inert additives and a 4H-3,1-benzoxazine derivative of the formula I as claimed in claim 1.

9. A process for combating unwanted plant growth, wherein the plants and/or their location are treated with a herbicidally effective amount of a 4H-3,1-benzoxazine derivative of the formula I as claimed in claim 1.

**Claims for the Contracting state: AT**

1. A fungicide containing inert additives and, as active ingredient, a 4H-3,1-benzoxazine derivative of the formula I

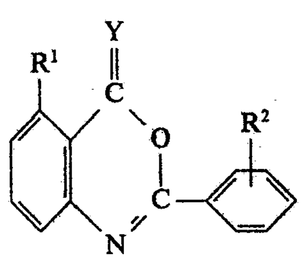

(I)

where Y is oxygen or sulfur, and $R^1$ is halogen if $R^2$ is halogen in the m- or p-position, or $R^1$ is methyl if $R^2$ is fluorine, trifluoromethyl, trifluoromethoxy or chlorodifluoromethoxy in the m-position, or $R^1$ is nitro if $R^2$ is trifluoromethoxy or chlorodifluoromethoxy in the m-position.

2. A herbicide containing inert additives and, as active ingredient, a 4H-3,1-benzoxazine derivative of the formula I as defined in claim 1, where Y is oxygen, $R^1$ is halogen and $R^2$ is halogen in the m- or p-position.

3. A herbicide containing inert additives and, as active ingredient, 2-(3'-fluorophenyl)-5-chloro-4H-3,1-benzoxazin-4-one.

4. A herbicide containing inert additives and, as active ingredient, 2-(3'-fluorophenyl)-5-fluoro-4H-3,1-benzoxazin-4-one.

5. A process for the manufacture of a 4H-3,1-benzoxazine derivative of the formula I as defined in claim 1, wherein a substituted anthranilic acid of the formula II

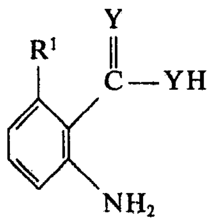

(II)

where $R^1$ and Y have the meanings given in claim 1, is reacted with not less than a two-fold molar excess of a carboxylic acid halide of the formula III

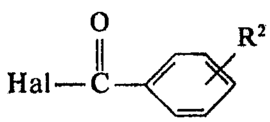

(III)

where $R^2$ has the meanings given in claim 1 and Hal is halogen, in an aromatic tertiary amine as the solvent, at from 10 to 60° C.

6. A process for the manufacture of a 4H-3,1-benzoxazine derivative of the formula I as defined in claim 1, wherein a substituted anthranilic acid of the formula II

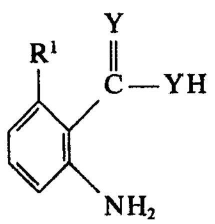

(II)

where $R^1$ and Y have the meanings given in claim 1, or an alkali metal salt or alkaline earth metal salt of this anthranilic acid, is reacted with about the stoichiometric amount of a carboxylic acid halide of the formula III

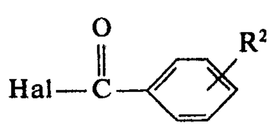

(III)

where $R^2$ has the meanings given in claim 1 and Hal is halogen, in an inert organic solvent or in water, in the presence or absence of an acid acceptor, at from 0 to 60° C, to give a carboxyamide of the formula IV

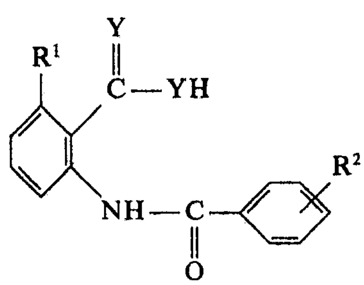

(IV)

where $R^1$, $R^2$ and Y have the meanings given in claim 1, and then the carboxamide is cyclized in the presence of a dehydrating agent at from 30 to 150° C.

**Revendications pour les Etats contractants: BE, CH, LI, DE, FR, GB, IT, LU, NL, SE**

1. Dérivés de 4H-3,1-benzoxazine de formule I

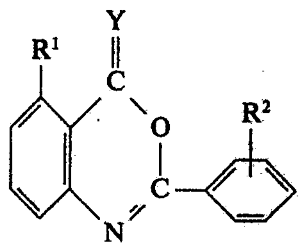

(I)

dans laquelle

Y représente oxygène ou soufre et
$R^1$ représente halogène, quand $R^2$ est halogène en position m- ou p, ou
$R^1$ représente méthyle, quand $R^2$ est fluor, trifluorométhyle, trifluorométhoxy ou chlorodifluorométhoxy en position m, ou
$R^1$ représente nitro, quand $R^2$ est trifluorométhoxy ou chlorodifluorométhoxy en position -m-.

2. Dérivés de 4H-3,1-benzoxazine de formule I selon la revendication 1, caractérisés par le fait que Y représente oxygène, $R^1$, halogène, et $R^2$, halogène en position -m- ou p-.

3. 2-(3'-fluorophényl)-5-chloro-4H-3,1-benzoxazin-4-one.

4. 2-(3'-fluorophényl)-5-fluoro-4H-3,1-benzoxazin-4-one.

5. Procédé de préparation de dérivés de 4H-3,1-benzoxazine de formule I selon la revendication 1, caractérisé par le fait qu'on fait réagir, dans une amine tertiaire aromatique, comme solvant, à une température comprise entre 10 et 60° C, un acide anthranilique substitué de formule II

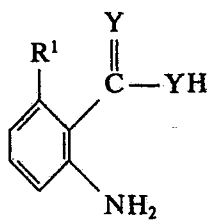

(II)

dans laquelle $R^1$ et Y ont les significations indiquées dans la revendications 1, avec au moins un excès molaire du double d'un halogénure d'acide carboxylique de formule III

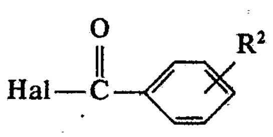

(III)

dans laquelle $R^2$ a la signification indiquée dans la revendication 1 et Hal représente halogène, dans une amine tertiaire aromatique, comme solvant, à une température comprise entre 10 et 60° C.

6. Procédé de préparation de dérivés de 4H-3,1-benzoxazine de formule I selon la revendication 1, caractérisé par le fait que l'on fait réagir un acide anthranilique substitué de formule II

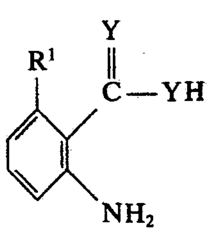

(II)

dans laquelle $R^1$ et Y ont les significations indiquées dans la revendication 1, ou un sel de métal alcalin ou alcalinoterreux de cet acide anthranilique avec une quantité à peu près stoechiométrique d'un

halogénure d'acide carboxylique de formule III

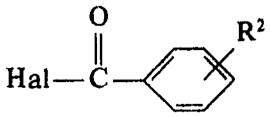

(III)

dans laquelle $R^2$ a la signification indiquée dans la revendication 1 et Hal représente halogène, dans un solvant organique inerte ou dans l'eau et éventuellement en présence d'un accepteur d'acide, à une température comprise entre 0 et 60° C, pour obtenir un carboxamide de formule IV

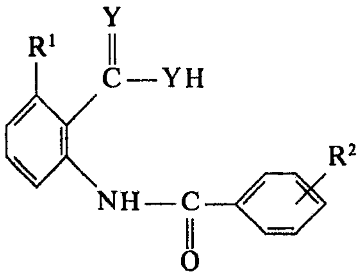

(IV)

dans laquelle $R^1$, $R^2$ et Y ont les signification indiquées dans la revendication 1, puis on cyclise ce composé en présence d'un agent déshydratant, à une température comprise entre 30 et 150° C.

7. Herbicide contenant un dérivé de 4H-3,1-benzoxazine de formule I selon la revendication 1.

8. Herbicide contenant un additif et un dérivé de 4H-3,1-benzoxazine de formule I selon la revendication 1.

9. Procédé de lutte contre la croissance indésirable des plantes, caractérisé par le fait que l'on traite les plantes et/ou leur habitat avec une quantité, acitive comme herbicide, d'un dérivé de 4H-3,1-benzoxazine de formule I selon la revendication 1.

**Revendications pour l'Etat contractant: AT**

1. Herbicide contenant des additifs inertes et, comme principe actif, un dérivé de 4H-3,1-benzoxazine de formule I

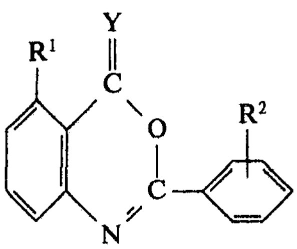

(I)

dans laquelle

Y représente oxygène ou soufre et
$R^1$ représente halogène, quand $R^2$ est halogène en position m- ou p, ou
$R^1$ représente méthyle, quand $R^2$ est fluor, trifluorométhyle, trifluorométhoxy ou chlorodifluorométhoxy en position m, ou
$R^1$ représente nitro, quand $R^2$ est trifluorométhoxy ou chlorodifluorométhoxy en position -m-.

2. Herbicide contenant des additifs inertes et, comme principe actif, un dérivé de 4H-3,1-benzoxazine de formule I selon la revendication 1, dans laquelle Y représente oxygène, $R^1$, halogène, et $R^2$, halogène en position m- ou p-.

3. Herbicide contenant des additifs inertes et, comme principe actif, de la 2-(3'-fluorophényl)-5-chloro-4H-3,1-benzoxazin-4-one.

4. Herbicide contenant des additifs inertes et, comme principe actif, de la 2-(3'-fluorophényl)-5-fluoro-4H-3,1-benzoxazin-4-one.

5. Procédé de préparation de dérivés de 4H-3,1-benzoxazine de formule I selon la revendication 1, caractérisé par le fait qu'on fait réagir, dans une amine tertiaire aromatique, comme solvant, à une température comprise entre 10 et 60° C, un acide anthranilique substitué de formule II

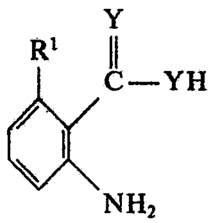

(II)

dans laquelle $R^1$ et Y ont les significations indiquées dans la revendication 1, avec au moins un excès molaire du double d'un halogénure d'acide carboxylique de formule III

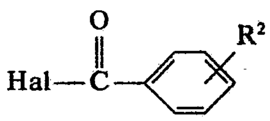

(III)

dans laquelle $R^2$ a la signification indiquée dans la revendication 1 et Hal représente halogène, dans une amine tertiaire aromatique, comme solvant, à une température comprise entre 10 et 60° C.

6. Procédé de préparation de dérivés de 4H-3,1-benzoxazine de formule I selon la revendication 1, caractérisé par le fait que l'on fait réagir un acide anthranilique substitué de formule II

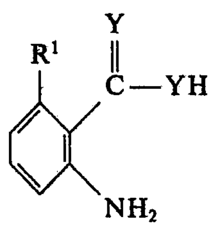

(II)

dans laquelle $R^1$ et Y ont les significations indiquées dans la revendication 1, ou un sel de métal alcalin ou alcalinoterreux de cet acide anthranilique avec une quantité à peu près stoechiométrique d'un halogène d'acide carboxylique de formule III

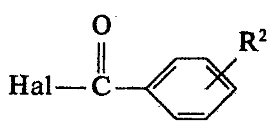

(III)

dans laquelle $R^2$ a la signification indiquée dans la revendication 1 et Hal représente halogène, dans un solvant organique inerte ou dans l'eau et éventuellement en présence d'un accepteur d'acide, à une température comprise entre 0 et 60° C, pour obtenir un carboxamide de formule IV

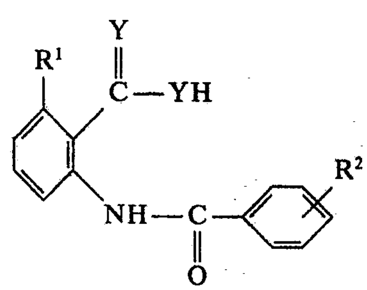

(IV)

dans laquelle $R^1$, $R^2$ et Y ont les significations indiquées dans la revendication 1, puis on cyclise ce composé en présence d'un agent déshydratant, à une température comprise entre 30 et 150° C.